# EUROPÄISCHE PATENTSCHRIFT

(11) **EP 2 637 766 B1**
(45) Veröffentlichungstag und Bekanntmachung des Hinweises auf die Patenterteilung: **17.06.2015**
(21) Anmeldenummer: 11782601.6
(22) Anmeldetag: 10.11.2011
(51) Int. Cl.: B01D 53/14, C07D 211/58, C10K 1/00, C10L 3/10, F23J 15/04

(54) **AMINHALTIGES ABSORPTIONSMEDIUM, VERFAHREN UND VORRICHTUNG ZUR ABSORPTION VON SAUREN GASEN AUS GASMISCHUNGEN**
AMINE CONTAINING ABSORPTION-MEDIUM, METHOD AND DEVICE FOR THE ABSORPTION OF ACID GASES FROM GAS MIXTURES
MILIEU ABSORBANT CONTENANT DES AMINES, MÉTHODE ET INSTALLATION POUR L'ABSORPTION DE GAZ ACIDES DE MELANGES GAZEUSES

(30) Priorität: 10.06.2011 DE 102011077377; 12.11.2010 DE 102010043838
(43) Veröffentlichungstag der Anmeldung: 18.09.2013
(73) Patentinhaber: Evonik Degussa GmbH, 45128 Essen (DE)
(72) Erfinder: SEILER, Matthias, 64347 Griesheim (DE); SCHNEIDER, Rolf, 63584 Gründau-Rothenbergen (DE); ROLKER, Jörn, 63755 Alzenau (DE); DEMBKOWSKI, Daniel, 45128 Essen (DE); NEUMANN, Manfred, 45770 Marl (DE); WITTHAUT, Daniel, 61273 Wehrheim (DE); KEUP, Michael, 45772 Marl (DE); BREHME, Volker, 48301 Nottuln (DE); IRFAN, Muhammad, 63526 Erlensee (DE)
(86) Internationale Anmeldenummer: PCT/EP2011/069787
(87) Internationale Veröffentlichungsnummer: WO 2012/062830

(56) Entgegenhaltungen:
- EP-A2- 0 302 020
- WO-A1-2010/089257

## Beschreibung

Die Erfindung beschreibt ein Verfahren zur Absorption von sauren Gasen aus einer Gasmischung, sowie Absorptionsmedien und eine Vorrichtung zur Durchführung dieses Verfahrens.

In zahlreichen industriellen und chemischen Prozessen treten Gasströme auf, die einen unerwünschten Gehalt von CO₂ und/oder anderen sauren Gasen aufweisen und deren Gehalt für die weitere Verarbeitung oder für den Transport minimiert bzw. entfernt werden muss.

Bei diesen Gasströmen handelt es sich beispielsweise um Erdgas, Synthesegas aus Schweröl, Raffineriegas oder verflüssigte Kohlenwasserstoffströme. Auch Reaktionsgase, die bei der partiellen Oxidation von organischen Materialien, wie beispielsweise Kohle oder Erdöl, entstehen, können CO₂ und/oder andere saure Gase aufweisen. Auch bei Biogasen ist der Gehalt an CO₂ und/oder sauren Gasen häufig unerwünscht, die aus vergärbaren, biomassehaltigen Reststoffen, wie beispielsweise Klärschlamm, Bioabfall, Speiseresten, Wirtschaftsdünger (Gülle, Mist), Pflanzenteilen sowie angebauten Energiepflanzen, entstehen können. Ähnlich verhält es sich mit Abgasen aus Verbrennungsprozessen, wie beispielsweise Rauchgase aus Kraftwerksprozessen. Aus diesen verschiedenen Gasströmen muss der Gehalt an CO₂ und/oder sauren Gasen aus den verschiedensten Gründen minimiert werden. Neben der Verminderung der Emission von Kohlendioxid, die als Hauptursache für den so genannten Treibhauseffekt angesehen wird, sind saure Gase häufig Katalysatorgifte in Folgeprozessen, tragen zur Korrosion bei oder mindern den Brennwert (z. B. bei Erdgas). Ein weiterer Aspekt ist, dass für einige Prozesse Kohlendioxid als Edukt benötigt wird.

Im industriellen Maßstab werden zur Absorption von CO₂ aus einer Gasmischung üblicherweise wässrige Lösungen von Alkanolaminen als Absorptionsmedium eingesetzt. Das beladene Absorptionsmedium wird durch Erwärmen, Entspannen auf einen niedrigeren Druck oder Strippen regeneriert, wobei das Kohlendioxid desorbiert wird. Nach dem Regenerationsprozess kann das Absorptionsmedium wieder verwendet werden. Diese Verfahren sind zum Beispiel in Rolker, J.; Arlt, W.; "Abtrerinung von Kohlendioxid aus Rauchgasen mittels Absorption" in Chemie Ingenieur Technik 2006, 78, Seiten 416 bis 424 sowie in Kohl, A. L.; Nielsen, R. B., "Gas Purification", 5. Aufl., Gulf Publishing, Houston 1997 beschrieben.

Diese Verfahren haben den Nachteil, dass zur Abtrennung von CO₂ durch Absorption und nachfolgende Desorption relativ viel Energie benötigt wird und dass bei der Desorption nur ein Teil des absorbierten CO₂ wieder desorbiert wird, so dass in einem Zyklus aus Absorption und Desorption der Anteil des zur Absorption von CO₂ genutzten Alkanolamins gering ist. Darüber hinaus sind die verwendeten Absorptionsmedien stark korrosiv und unterliegen bei der Absorption von CO₂ aus sauerstoffhaltigen Gasmischungen einem störenden oxidativen Abbau.

US 7,419,646 beschreibt ein Verfahren zur Entsäuerung von Abgasen, bei dem ein Absorptionsmedium verwendet wird, das bei der Absorption des sauren Gases zwei voneinander trennbare Phasen ausbildet. Als reaktive Verbindung zur Absorption eines sauren Gases wird in Spalte 6 unter anderem 4-Amino-2,2,6,6-tetramethylpiperidin genannt. Das Verfahren von US 7,419,646 hat den Nachteil, dass zusätzliche Apparate für die Trennung der bei der Absorption anfallenden zwei Phasen erforderlich sind.

DD 266 799 beschreibt ein Verfahren zur Reinigung von 4-Amino-2,2,6,6-tetramethylpiperidin, bei dem CO₂ in eine Lösung von 4-Amino-2,2,6,6-tetramethylpiperidin in Wasser und Aceton eingeleitet wird und das ausgefällte Salz durch Erhitzen auf 90 bis 200°C wieder zu CO₂ und 4-Amino-2,2,6,6-tetramethylpiperidin zersetzt wird.

WO 2010/089257 beschreibt ein Absorptionsmedium für die Absorption von CO₂ aus einer Gasmischung, wobei das Absorptionsmedium Wasser und mindestens ein 4-(Dialkylamino)-2,2,6,6-tetramethylpiperidin oder 4-Amino-2,2,6,6-tetramethylpiperidin aufweist. Der Einsatz von 4-Amino-2,2,6,6-tetramethylpiperidin hat häufig den Nachteil, dass sich diese Verfahren durch geringere CO₂-Hübe auszeichnen, dadurch bedingt, dass ein größerer Massenstrom an Absorptionsmedium im Verfahren gefördert und auch im Desorber wieder regeneriert werden muss.

WO 2009/156271 beschreibt ein Absorptionsmedium für die Absorption von Sauergasen aus Fluidströmen, insbesondere aus Rauchgasen. Das Absorptionsmedium weist hierfür ein Oligoamin und ein primäres oder sekundäres Alkanolamin auf. Das 4-Amino-2,2,6,6-tetramethylpiperidin kann dem Absorptionsmedium als Aktivator zugesetzt sein. Die primären Amine weisen in der Regel hohe Absorptionsenthalpien auf, dadurch bedingt sind höhere Verdampferleistungen bei der Desorption.

WO 2008/015217 beschreibt ein Verfahren zum Abtrennen von CO₂ aus Gasmischungen sowie eine entsprechende Vorrichtung hierfür. Hierfür wird ein absorbierendes Mittel mit mindestens einem sekundären und / oder mindestens tertiären Amin eingesetzt, als sekundäres Amin wird u.a. 2,2,6,6-Tetramethylpiperidin gelistet. Auch hier sind bedingt durch geringere CO₂-Hübe, größere Massenströme an Absorptionsmedium im Verfahren zu fördern, die auch im Desorber wieder regeneriert werden müssen. Ferner besteht die Gefahr, dass es nach der Absorption zu einer Ausfällung kommen kann.

Das Institut Francais du Petrole beschreibt in den Publikationen US 2009/0199709, FR 2900841 und US 2007/0286783 ein Absorptionsmedium, welches u. a. 4-Amino-2,2,6,6-tetramethylpiperidin und/oder 1,2,2,6,6-Pentamethyl-4-piperidin als Amin aufweist. Das 4-Amino-2,2,6,6-tetramethylpiperidin kann dem Absorptionsmedium auch als Aktivator zugesetzt werden. Hier ergeben sich dieselben Nachteile wie sie bereits für die WO 2010/089257 genannt sind.

EP 0 302 020 A beschreibt ein Verfahren zur Herstellung von 2,2,6,6-Tetramethyl-4-piperidylaminen sowie deren Verwendung zur Herstellung von Lichtstabilisatoren für Polymere.

Es war daher die Aufgabe der vorliegenden Erfindung ein verbessertes und somit wirtschaftlicheres Verfahren zur Verfügung zu stellen.

Überraschenderweise wurde ein Verfahren zur Absorption von sauren Gasen aus einer Gasmischung durch in Kontakt bringen der Gasmischung mit einem Absorptionsmedium gefunden, welches sich dadurch auszeichnet, dass das Absorptionsmedium ein Amin (A) der Formel (I) aufweist. Das erfindungsgemäße Absorptionsmedium zeichnet sich gegenüber den Verfahren gemäß dem Stand der Technik durch seine verbesserte Fähigkeit saure Gase, insbesondere CO_{2;} zu binden aus, so dass insgesamt ein größerer CO₂-Hub erreicht werden kann. Dies bietet die Möglichkeit, die Anlagenbauteile in dem erfindungsgemäßen Verfahren kleiner dimensionieren zu können. So können beispielsweise Pumpen, Behälter, Rohrleitungen und Absorptions- bzw. Desorptionskolonnen mit einem kleineren Durchmesser in dem erfindungsgemäßen Verfahren eingesetzt werden. Aufgrund der Eigenschaften des erfindungsgemäßen Absorptionsmediums können auch geringere Mengen an Absorptionsmedium in dem erfindungsgemäßen Verfahren eingesetzt werden. Somit können auch die Puffertankbauteile kleiner dimensioniert werden. Auf Grund der verringerten Mengen an eingesetztem Absorptionsmedium kann auch der Energieverbrauch reduziert werden, da weniger Energie für den Verfahrensschritt Desorption notwendig ist.

Weiterhin ist die aufzubringende Energie zur Abspaltung des absorbierten CO₂ geringer als gemäß dem Stand der Technik. Darüber hinaus konnte völlig unerwartet ein Synergieeffekt beobachtet werden, wenn ein Absorptionsmedium einsetzt wird, das sowohl ein Amin (A) der Formel (I) als auch ein Amin (B) der Formel (III) aufweist. Der hierbei gemessene CO₂-Hub ist größer als das arithmetische Mittel der gemessenen CO₂-Hübe für die Absorptionsmedien mit jeweils nur einem dieser Amine.

Gegenstand der Erfindung ist somit ein Verfahren zur Absorption eines sauren Gases aus einer Gasmischung durch in Kontakt bringen der Gasmischung mit einem Absorptionsmedium, das dadurch gekennzeichnet ist, dass ein Absorptionsmedium eingesetzt wird, das zumindest Wasser als Lösemittel und mindestens ein Amin (A) der Formel (I) 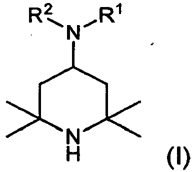 mit R¹ = aliphatischer Rest mit 2 bis 6 Kohlenstoffatomen und mit mindestens einer Aminogruppe,
R² = Wasserstoff, ein C₁₋₄-Alkylgruppe oder ein Rest R¹
aufweist.

Des Weiteren ist Gegenstand der Erfindung ein Absorptionsmedium, das zumindest Wasser als Lösemittel, mindestens ein Amin (A) der Formel (I) und zusätzlich ein weiteres Amin (B) der Formel (III) 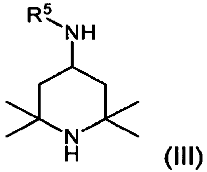 mit R⁵ = C₁₋₆-Alkylgruppe, bevorzugt Butylgruppe, aufweist, sowie eine Vorrichtung zur Abtrennung von sauren Gasen aus einer Gasmischung, umfassend eine Absorptionseinheit, eine Desorptionseinheit und ein im Kreislauf geführtes Absorptionsmedium, welche sich dadurch auszeichnet, dass sie ein erfindungsgemäßes Absorptionsmedium umfasst.

Das erfindungsgemäße Verfahren zur Absorption eines sauren Gases aus einer Gasmischung durch in Kontakt bringen der Gasmischung mit einem Absorptionsmedium zeichnet sich dadurch aus, dass ein Absorptionsmedium eingesetzt wird, das zumindest Wasser als Lösemittel und mindestens ein Amin (A) der Formel (I) 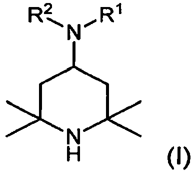 mit R¹ = aliphatischer Rest mit 2 bis 6 Kohlenstoffatomen und mit mindestens einer Aminogruppe,
R² = Wasserstoff, C₁₋₄-Alkylgruppe oder Rest R¹,
aufweist. Vorzugsweise weist das eingesetzte Absorptionsmedium ein Amin (A) der Formel (I) auf, worin R² Wasserstoff ist.

Bevorzugt weist das eingesetzte Absorptionsmedium ein Amin (A) der Formel (II) 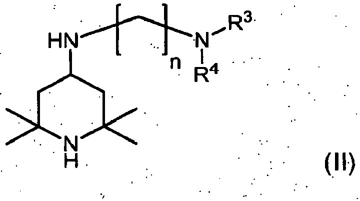 mit R³ = Wasserstoff oder C₁₋₄-Alkylgruppe, bevorzugt Wasserstoff oder Methylgruppe, besonders bevorzugt Methylgruppe,
R⁴ = C₁₋₄-Alkylgruppe, bevorzugt C₁₋₂-Alkylgruppe, besonders bevorzugt Methylgruppe,
n = 2 bis 4, bevorzugt 2 bis 3 und besonders bevorzugt 3,
auf.

Besonders bevorzugt weist das eingesetzte Absorptionsmedium ein Amin (A) der Formel (II) mit R³, R⁴ = Methyl und n = 3 auf.

Die Alkylgruppen können im Rahmen dieser Erfindung substituiert oder unsubstituiert sein, ferner können C₃₋₄-Alkylgruppen linear oder verzweigt sein.

Das in dem erfindungsgemäßen Verfahren eingesetzte Absorptionsmedium kann neben dem Amin (A) ein weiteres Amin (B) der Formel (III) 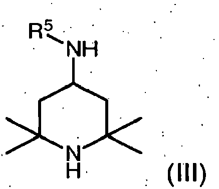 mit R⁵ = C₁₋₆-Alkylgruppe, bevorzugt C₃₋₅-Alkylgruppe, und besonders bevorzugt Butylgruppe,
aufweisen.

Die Alkylgruppen können in Rahmen dieser Erfindung substituiert oder unsubstituiert sein, ferner können die C₃₋₆-Alkylgruppen linear oder verzweigt sein. Der Substituent vom Typ R⁵ ist vorzugsweise unsubstituiert, bevorzugt eine unsubstituierte Butylgruppe und besonders bevorzugt eine unsubstituierte n-Butylgruppe.

Das in dem erfindungsgemäßen Verfahren eingesetzte Absorptionsmedium weist zumindest Wasser als Lösemittel auf. Das eingesetzte Absorptionsmedium kann jedoch noch ein weiteres physikalisches Lösemittel (C) enthalten. Dies ist von Vorteil, um die Beladung des Absorptionsmediums mit den sauren Gasen bei hohem Partialdruck des sauren Gases, insbesondere von CO₂, noch weiter zu erhöhen. Auf diese Weise kann der Massenstrom an Absorptionsmedium nochmals reduziert werden. Gleichzeitig lässt sich eine energetisch günstigere Regeneration durchführen, indem nicht mehr nur thermisch, sondern alternativ bzw. unterstützend auch durch einen Flash (Druckerniedrigung) regeneriert werden kann.

Die Auswahl des Lösemittels (C) und der Gehalt des Lösemittels (C) in dem eingesetzten Absorptionsmittels richtet sich nach verschiedenen Kriterien, wie beispielsweise der Zusammensetzung der zu reinigenden Gasmischung (z.B. Anteile an sauren Komponenten, Anteil an Kohlenwasserstoffen), dem vorliegenden Partialdruck der zu entfernenden sauren Gase, wie beispielsweise CO₂, sowie auch den zu erfüllenden Spezifikationen an das mittels dem erfindungsgemäßen Verfahren gereinigte Gas.

In einer besonderen Ausführungsform des erfindungsgemäßen Verfahrens beträgt der Gehalt an dem Lösemittel (C) bezogen auf das eingesetzte Absorptionsmedium von 20 bis 40 Gew.-%. Diese Ausführungsform eignet sich insbesondere, wenn der Partialdruck des sauren Gases besonders hoch ist, vorzugsweise mindestens 20 bar beträgt, und die Anforderungen an das gereinigte Gas ebenfalls hoch sind, vorzugsweise wenn der Partialdruck des sauren Gases maximal 10 mbar im gereinigten Gas betragen soll. Auf diese Weise kann die Beladung des Absorptionsmediums mit den sauren Gasen nochmals erhöht werden, dies führt wiederum zu niedrigeren Massenströmen an Absorptionsmedium. Der Anteil an dem Amin (A) beträgt vorzugsweise 10 bis 25 Gew.-% und der Anteil an dem Amin (B) vorzugsweise 5 bis 15 Gew.% bezogen auf das eingesetzte Absorptionsmedium. Auf diese Weise ist eine Aufreinigung eines Gases möglich mit dem Ziel einen möglichst geringen Partialdruck des sauren Gases zu erzielen. Weiterhin vermindert sich der eingesetzte Massenstrom an Absorptionsmedium. Durch den verringerten Massenstrom sowie den Anteil des physikalischen Lösungsmittels kann eine energetisch günstigere Regeneration des Lösungsmittels erreicht werden, denn ein Teil des Sauergases kann über einen Flash abgetrennt werden. Zusätzlich kann noch durch einen Desorber regeneriert werden, um das Lösungsmittel weiter an Sauergasen abzureichern, jedoch bei verringertem Dampfbedarf im Verdampfer des Desorbers.

Als weiteres Lösemittel (C) kann das in dem erfindungsgemäßen Verfahren eingesetzte Absorptionsmedium die aus der Gaswäsche bekannten physikalischen Lösemittel, wie beispielsweise Sulfolan, Propylencarbonat, N-alkylierte Pyrrolidone (z.B. N-Methyl-2-pyrrolidone), und N-alkylierte Piperidone, Dialkylether von Polyethylenglykol und Mischungen daraus, aliphatische Säureamide (z.B. N-Formylmorpholin oder N-Acetylmorpholin), Methylcyanoacetat, aufweisen.

Das weitere Lösemittel (C) kann in dem erfindungsgemäßen Verfahren auch die Wirkung eines Lösungsvermittlers aufweisen. Durch den Zusatz eines weiteren Lösemittels (C) kann die Temperatur, bei der eine Phasentrennung des mit sauren Gasen beladenen Absorptionsmittels stattfindet, heraufgesetzt werden. Dies vereinfacht insbesondere die anschließende Desorption, da hier häufig auch mit Temperaturerhöhung gearbeitet wird, und somit keine besonderen verfahrenstechnische Maßnahmen für ein Mehrphasensystem notwendig sind. Die Temperatur, bei der die Phasentrennung einsetzt, ist bei einem Absorptionsmedium, das ausschließlich Wasser und ein Amin (A) der Formel (I) aufweist, höher als bei einem Absorptionsmittel, das ausschließlich Wasser und ein Amin (B) der Formel (III) aufweist. Das erfindungsgemäße Verfahren hat daher den Vorteil, dass die Desorption der sauren Gase bzw. die Regeneration des Absorptionsmediums bei höheren Temperaturen erfolgen kann, ohne dass eine Phasentrennung auftritt. Je nach Erfordernissen der Anwendung kann durch die entsprechend gewählte Formulierung des Absorptionsmediums gemäß bestimmter Anteile der Amine (A), (B) und des Lösungsmittels (C) ein favorisiertes Regenerationsverfahren gewählt werden. Wenn z.B. ein hoher Abreicherungsgrad an den Sauergasen erzielt werden muss und ein bestehendes Anlagenequipment mit Desorberkolonne verwendet werden soll, kann eine Zusammensetzung gewählt werden, bei der sich das mit sauren Gasen beladene Absorptionsmedium nicht entmischt.

Ferner kann die Zusammensetzung des Absorptionsmediums derart gewählt werden, dass sich bei einer Temperaturanhebung eine Entmischung des mit sauren Gasen beladenen Absorptionsmittels in eine wässrige und eine organische Phase einstellt. Dieser Fall kann weitere Vorteile bei der Regeneration bieten, da bereits ein energetisch günstigerer Flash ausreichend ist und das mit sauren Gasen beladene Absorptionsmittel in einem Flash bei moderater Temperaturerhöhung vom sauren Gas, insbesondere CO₂, befreit werden kann.

Vorzugsweise wird in dem erfindungsgemäßen Verfahren ein Absorptionsmedium eingesetzt, das
60 bis 80 Gew.-% an Wasser und ggf. Lösemittel (C),
1 bis 40 Gew.-% an dem Amin (A) und
0 bis 39 Gew.-% an dem Amin (B)
aufweist. Bevorzugt weist das eingesetzte Absorptionsmedium
65 bis 75 Gew.-% an Wasser und ggf. Lösemittel (C),
10 bis 20 Gew.-% an dem Amin (A) und
25 bis 5 Gew.-% an dem Amin (B)
auf.

Besonders bevorzugt weist das eingesetzte Absorptionsmedium
65 bis 75 Gew.-% an Wasser,
10 bis 20 Gew.-% an dem Amin (A) Forme) (II) mit R³, R⁴ = Methyl und n = 3 und
25 bis 5 Gew.-% an Amin (B) der Formel (III) mit R⁵ = n-Propyl oder n-Butyl
auf. Mit dieser Zusammensetzung kann für das mit sauren Gasen beladene Absorptiönsmittel eine Entmischungstemperatur im Bereich von 85 bis 110 °C eingestellt werden.

Das in dem erfindungsgemäßen Verfahren eingesetzten Absorptionsmedium kann auch Performanceadditive, wie beispielsweise Korrosionsinhibitoren, Aktivatoren, benetzungsfördernde Additive und/oder Entschäumer aufweisen.

Als Korrosionsinhibitoren können im erfindungsgemäßen Verfahren alle Stoffe verwendet werden, die dem Fachmann für Verfahren zur Absorption von CO₂ unter Verwendung von Alkanolaminen als geeignete Korrosionsinhibitoren bekannt sind, insbesondere die in US 4,714,597 beschriebenen Korrosionsinhibitoren.

Die Menge an Korrosionsinhibitoren, die das in dem erfindungsgemäßen Verfahren eingesetzte Absorptionsmedium vorzugsweise aufweist, ist gegenüber Verfahren gemäß dem Stand der Technik deutlich reduziert, da das in dem erfindungsgemäßen Verfahren eingesetzte Absorptionsmedium gegenüber metallischen Werkstoffen deutlich weniger korrosiv ist als das gemäß dem Stand der Technik häufig eingesetzte Monoethanolamin.

Als benetzungsförderndes Addditiv werden vorzugsweise ein oder mehrere Tenside aus der Gruppe der nichtionischen Tenside, zwitterionischen Tenside und kationischen Tenside verwendet.

Geeignete nichtionische Tenside sind Alkylaminalkoxylate, Amidoamine, Alkanolamide, Alkylphosphinoxide, Alkyl-N glucamide, Alkylglucoside, Gallensäuren, Alkylalkoxylate, Sorbitanester, Sorbitanesterethoxylate, Fettalkohole, Fettsäureethoxylate, Esterethoxylate und Polyethersiloxane.
Geeignete zwitterionischen Tenside sind Betaine, Alkylglycine, Sultaine, Amphopropionate, Amphoacetate, tertiäre Aminoxide und Silicobetaine.

Geeignete kationische Tenside sind quaternäre Ammoniumsalze mit einem oder zwei Substituenten mit 8 bis 20 Kohlenstoffatomen, insbesondere entsprechende Tetraalkylammoniumsalze, Alkylpyridiniumsalze, Esterquats, Diamidoaminquats, Imidazoliniumquats, Alkoxyalkylquats, Benzylquats und Silikonquats.

In einer bevorzugten Ausführungsform umfasst das benetzungsfördernde Additiv ein oder mehrere nichtionische Tenside der allgemeinen Formel R(OCH₂CHR')ₘOH mit m von 4 bis 40, worin R ein Alkylrest mit 8 bis 20 Kohlenstoffatomen, ein Alkylarylrest mit 8 bis 20 Kohlenstoffatomen oder ein Polypropylenoxidrest mit 3 bis 40 Propylenoxideinheiten ist und R' Methyl oder vorzugsweise Wasserstoff ist.

In einer weiteren bevorzugten Ausführungsform umfasst das benetzungsfördernde Additiv ein Polyether-Polysiloxan-Copolymer, das mehr als 10 Gew.-% [Si(CH₃)₂O]-Einheiten und mehr als 10 Gew.-% [CH₂CHR"-O]-Einheiten enthält, in denen R" Wasserstoff oder Methyl ist. Besonders bevorzugt sind Polyether-Polysiloxan-Copolymere der allgemeinen Formeln (IV) bis (VI):

(CH₃)₃Si-O-[SiR^{a}(CH₃)-O]ₜ-Si(CH₃)₃ (IV)

R^{b}O-Aₚ-[B-A]ₘ-A_{q}-R^{b} (V)

R^{b}O-[A-Z]ₚ-[B-Si(CH₃)₂-Z-O-A-Z]ₘ-B-Si(CH₃)₂[Z-O-A]_{q}O_{1-q}R^{b} (VI)

worin
- A: ein zweiwertiger Rest der Formel -[CH₂CHR^{c}-O]ᵣ ist,
- B: ein zweiwertiger Rest der Formel-[Si(CH₃)₂-O]ₛ- ist,
- Z: ein zweiwertiger linearer oder verzweigter Alkylenrest mit 2 bis 20 Kohlenstoffatomen und vorzugsweise -(CH₂)₃- ist,
- t: = 1 bis 30 ist,
- m: = 2 bis 100 ist,
- p, q: = 0 oder 1 ist,
- r: = 2 bis 100 ist,
- s: = 2 bis 100 ist,
- R^{a}: von 1 bis 5 der Reste R^{a} Reste der allgemeinen Formel -Z-O-A-R^{b}- sind und die restlichen Reste R² Methyl sind,
- R^{b}: Wasserstoff, ein Alkylrest oder ein aliphatischer oder olefinischer Acylrest mit 1 bis 20 Kohlenstoffatomen ist und
- R^{c}: Wasserstoff oder Methyl ist.

Die benetzungsfördernden Additive sind dem Fachmann bereits aus dem Stand der Technik als Additive für wässrige Lösungen bekannt und können nach aus dem Stand der Technik bekannten Verfahren hergestellt werden.

Das in dem erfindungsgemäßen Verfahren eingesetzte Absorptionsmedium kann sogenannte Aktivatoren aufweisen. Durch den Einsatz von Aktivatoren kann die gewünschte Trennwirkung noch weiter verbessert werden. Als Aktivatoren werden in dem erfindungsgemäßen Verfahren vorzugsweise primäre oder sekundäre Amine eingesetzt, wobei diese keine Struktur gemäß Formel (I) bis (III) aufweisen. Es eignen sich hierfür vorzugsweise Amine, die eine schnelle Kinetik hinsichtlich der Bindung der sauren Gase, insbesondere des CO₂, aufweisen. Bevorzugt werden Aktivatoren ausgewählt aus Monoethanolamin, Piperazin und 3-(Methylamino)propylamin eingesetzt. Das Absorptionsmedium weist in dem erfindungsgemäßen Verfahren vorzugsweise von 0 bis 20 Gew.-% an den Aktivatoren auf.

Als saure Gase sind Verbindungen zur verstehen, die in der zu reinigenden Gasmischung unter den vorliegenden Bedingungen gasförmig vorliegen und in wässriger Lösung einen pH-Wert von kleiner 7 aufweisen. Typische saure Gase sind beispielsweise Kohlendioxid (CO₂), Schwefelwasserstoff (H₂S), Schwefeldioxid (SO₂), Kohlenstoffoxidsulfid (COS), Kohlenstoffdisulfid (CS₂), Cyanwasserstoff (HCN) und Mercaptane (RSH). Das erfindungsgemäße Verfahren wird vorzugsweise zur Entfernung von CO₂ aus einer Gasmischung eingesetzt.

Mittels dem erfindungsgemäßen Verfahren können Gasmischungen, ausgewählt aus Erdgas, Synthesegas, Verbrennungsabgase; Abgase aus biologischen Prozessen wie Kompostierungen, Fermentationen oder Kläranlagen; Abgase aus Kalzinierprozessen, wie Kalkbrennen und der Zementherstellung; Restgase aus Hochofenprozessen der Eisenherstellung; sowie Restgase aus chemischen Verfahren, sowie Abgase der Russherstellung oder der Wasserstoffherstellung durch Dampfreformierung, gereinigt werden, wobei die sauren Gase, insbesondere CO₂, entfernt werden.

Das erfindungsgemäße Verfahren eignet sich vorzugsweise für die Entfernung von CO₂ aus Erdgas, Synthesegas, Rauchgasen oder einem Verbrennungsabgas, bevorzugt für die Entfernung von CO₂ aus Erdgas, Synthesegas oder einem Verbrennungsabgas.

Besonders bevorzugt wird als Gasmischung Erdgas oder Synthesegas in dem erfindungsgemäßen Verfahren eingesetzt.

Der Restgehalt an CO₂ des mittels dem erfindungsgemäßen Verfahren gereinigten Gases beträgt bei Pipeline-Erdgas vorzugsweise maximal 2 Gew.-%, bei Flüssigerdgas vorzugsweise maximal 50 ppm und bei Synthesegas vorzugsweise maximal 500 ppm.

Für das erfindungsgemäße Verfahren können alle zum in Kontakt bringen einer Gasphase mit einer Flüssigphase geeigneten Apparate verwendet werden, um die Gasmischung mit dem Absorptionsmedium in Kontakt zu bringen. Vorzugsweise werden aus dem Stand der Technik bekannte Gaswäscher oder Absorptionskolonnen verwendet, beispielsweise Membrankontaktoren, Radialstromwäscher, Strahlwäscher, Venturi-Wäscher, Rotations-Sprühwäscher, Füllkörperkolonnen, Packungskolonnen und Bodenkolonnen. Besonders bevorzugt werden Absorptionskolonnen im Gegenstrombetrieb verwendet.

Im erfindungsgemäßen Verfahren wird die Absorption der sauren Gase, insbesondere von CO₂, vorzugsweise bei einer Temperatur des Absorptionsmediums im Bereich von 0 bis 70 °C und bevorzugt 20 bis 60 °C, durchgeführt. Bei Verwendung einer Absorptionskolonne im Gegenstrombetrieb beträgt die Temperatur des Absorptionsmediums besonders bevorzugt 30 bis 60 °C beim Eintritt in die Kolonne und 35 bis 70°C beim Austritt aus der Kolonne.

In dem erfindungsgemäßen Verfahren ist der Gesamtdruck der Gasmischung bei dem Verfahrensschritt der Absorption von geringerer Bedeutung. Es hat sich jedoch als vorteilhaft herausgestellt, dass die Absorption von sauren Gasen, insbesondere von CO₂) bei einem Gesamtdruck der Gasmischung im Bereich von 0,8 bis 50 bar, bevorzugt im Bereich von 0,9 bis 30 bar, durchgeführt wird. In einer besonders bevorzugten Ausführungsform wird die Absorption bei einem Gesamtdruck der Gasmischung im Bereich von 0,8 bis 1,5 bar, insbesondere 0,9 bis 1,1 bar, durchgeführt. Diese besonders bevorzugte Ausführungsform ist bei der Absorption von CO₂ aus dem Verbrennungsabgas eines Kraftwerks ohne Verdichtung des Verbrennungsabgases empfehlenswert.

Der Partialdruck des sauren Gases, insbesondere des CO₂ variiert mit der mittels des erfindungsgemäßen Verfahrens zu reinigenden Gasmischung. So beträgt der Partialdruck der sauren Gase in der zu reinigenden Gasmischung von 0,1 bar bis 60 bar bei Erdgas, von 0,1 bar bis 35 bar bei Synthesegas und von 0,03 bar bis 0,15 bar bei Rauchgasen aus Kraftwerken.

In einer besonders bevorzugten Ausführungsform des erfindungsgemäßen Verfahrens beträgt der Partialdruck des sauren Gases, insbesondere des CO₂, von 0,1 bar bis 20 bar.

In einer weiteren bevorzugten Ausführungsform des erfindungsgemäßen Verfahrens liegen besonders hohe Partialdrücke des sauren Gases, insbesondere des CO₂, in der Gasmischung vor, insbesondere Partialdrücke von mindestens 15 bar. In diesem Fall beträgt der Anteil des physikalischen Lösemittets (C) in dem Absorptionsmedium mindestens 30 Gew.-%.

Nach dem Verfahrensschritt der Absorption kann das den Absorber verlassende mit dem sauren Gas, insbesondere mit CO₂, beladene Absorptionsmedium ein- oder zweiphasig sein. Im erfindungsgemäßen Verfahren werden jedoch Temperatur und Druck in dem Verfahrensschritt der Absorption sowie die Zusammensetzung des Absorptionsmediums vorzugsweise so gewählt, dass das Absorptionsmedium nach der Absorption des sauren Gases, insbesondere von CO₂, einphasig vorliegt, d.h. die Absorption des sauren Gases im Absorptionsmedium führt nicht zur Ausfällung eines Feststoffs oder zur Ausbildung von zwei flüssigen Phasen. Diese bevorzugte Ausführungsform des erfindungsgemäßen Verfahrens erfordert daher keine zusätzlichen Apparate für eine Phasentrennung und kann in den aus dem Stand der Technik bekannten Vorrichtungen zur Absorption von CO₂ mit Alkanolaminen durchgeführt werden.

In einer bevorzugten Ausführungsform des erfindungsgemäßen Verfahrens wird im Absorptionsmedium absorbiertes saures Gas, insbesondere CO₂, durch Erhöhen der Temperatur und/oder durch Verringern des Drucks wieder desorbiert und das Absorptionsmedium nach dieser Desorption des sauren Gases, insbesondere von CO₂, wieder zur Absorption von sauren Gasen verwendet. Durch einen solchen zyklischen Prozess aus Absorption und Desorption können die sauren Gase, insbesondere CO₂, aus der Gasmischung ganz oder teilweise abgetrennt und getrennt von anderen Komponenten der Gasmischung erhalten werden.

Alternativ oder ergänzend zu einer Temperaturerhöhung und/oder Druckverringerung kann auch eine Desorption durch Strippen des mit sauren Gasen, insbesondere CO₂, beladenen Absorptionsmediums mit einem Gas, beispielsweise Stickstoff oder Luft, durchgeführt werden. Eine Desorption durch Strippen mit einem Gas hat gegenüber einer Desorption in einer Desorptionskolonne den Vorteil eines geringeren Energiebedarfs.

Wenn bei der Desorption von den sauren Gasen zusätzlich auch Wasser aus dem Absorptionsmedium entfernt wird, kann dem Absorptionsmedium vor der Wiederverwendung zur Absorption gegebenenfalls noch Wasser zugesetzt werden.

Für die Desorption können alle Apparate verwendet werden, die aus dem Stand der Technik zur Desorption eines Gases aus einer Flüssigkeit bekannt sind. Vorzugsweise wird die Desorption in einer Desorptionskolonne durchgeführt. Alternativ oder ergänzend kann die Desorption der sauren Gase, insbesondere von CO₂, auch in einer oder mehreren Flash-Verdampfurigsstufen durchgeführt werden.

Bei einer Desorption durch Temperaturerhöhung wird die Desorption der sauren Gase, insbesondere von CO₂, vorzugsweise bei einer Temperatur des Absorptionsmediums im Bereich von 50 bis 200°C, bevorzugt 55 bis 150°C und besonders bevorzugt von 60 bis 100 °C, durchgeführt. Die Temperatur bei der Desorption liegt dabei vorzugsweise mindestens 20 °C, besonders bevorzugt mindestens 50 °C, oberhalb der Temperatur bei der Absorption.

Bei dem Verfahrensschritt der Desorption in dem erfindungsgemäßen Verfahren wird durch Verringern des Drucks die Desorption der sauren Gase, insbesondere von CO₂, vorzugsweise bei einem Gesamtdruck in der Gasphase im Bereich von 0,01 bis 10 bar, insbesondere 0,1 bis 5 bar, durchgeführt. Bevorzugt wird die Desorption bei einem Druck von mindestens bei 1,5 bar und besonders bevorzugt von mindestens bei 2 bar durchgeführt.

Bei einer Desorption durch Erhöhen der Temperatur kann der Druck bei der Desorption der sauren Gase, insbesondere von CO₂, auch höher sein als bei der Absorption der sauren Gase. Bei dieser Ausführungsform liegt der Druck bei der Desorption der sauren Gase vorzugsweise bis zu 5 bar, besonders bevorzugt bis zu 3 bar oberhalb des Drucks bei der Absorption der sauren Gase. Mit dieser Ausführungsform können die aus der Gasmischung abgetrennten sauren Gase ohne Einsatz von mechanischer Energie auf einen höheren Druck als den der Gasmischung verdichtet werden.

In einer bevorzugten Ausführungsform des erfindungsgemäßen Verfahrens wird das mit sauren Gasen beladene Absorptionsmedium zuerst durch Druckerniedrigung in einer oder mehreren aufeinanderfolgenden Flash-Verdampfungsstufen von den sauren Gasen befreit und anschließend wird der noch verbliebene Anteil an sauren Gasen in einer Desorptionskolonne durch Strippen, bevorzugt mit einem Inertgas, wie beispielsweise Luft oder Stickstoff, entfernt. In der letzten Flash-Verdampfungsstufen kann der Druck bis auf 1 bis 5 bar, vorzugsweise auf 1 bis 2 bar abgesenkt werden. Diese Ausführungsform hat den Vorteil, dass die Temperatur im Desorber niedriger gewählt werden kann, vorzugsweise von 60 bis 100°C. Ferner kann durch die Kombination von Druckerniedrigungen und Temperaturerhöhung das Absorptionsmittel effektiv von den sauren Gasen befreit werden und steht mit nahezu der gleichen geringen CO₂-Beladung für die nächste Absorption von sauren Gasen zu Verfügung. Auf diese Weise kann die Menge an Absorptionsmedium im Gesamtprozess gesenkt werden.

In einer weiteren Ausführungsform des erfindungsgemäßen Verfahrens bildet sich nach der Absorption abhängig von der Temperatur und der Zusammensetzung des Absorptionsmediums ein zweiphasiges flüssiges System aus. Die Desorption der sauren Gase bzw. Regeneration des Absorptionsmediums kann in diesem Fall auch durch eine Flash-Verdampfungsstufe oder durch mehrere aufeinanderfolgende Flash-Verdampfungsstufen erfolgen. Vor der Druckerniedrigung im Flash wird dabei die Temperatur des Absorptionsmittels angehoben. Auf diese Weise können die sauren Gase, wie beispielsweise CO₂ wieder aus dem Absorptionsmedium entfernt werden. Diese Art der Regeneration ist energetisch deutlich günstiger als der Betrieb einer Desorptiönskolonne. In diesem Fall empfiehlt es sich im weiteren Prozessverlauf vor dem Absorber geeignete Maßnahmen zu treffen, um das Absorptionsmedium wieder in eine homogene Lösung zu bringen. Hierfür eignen sich u. a. statische Mischer oder Behälter mit Rührer oder Umpumpvorrichtungen.

Vorzugsweise wird das Absorptionsmedium nach dem in Kontakt bringen mit der Gasmischung auf eine Temperatur erhitzt, bei der eine Phasentrennung in eine wässrige flüssige Phase und eine organische flüssige Phase erfolgt und aus der resultierenden zweiphasigen Mischung wird saures Gas durch Strippen mit einem Inertgas desorbiert. Als Inertgas eignen sich dabei alle Gase, die unter den Bedingungen der Desorption keine Reaktion mit den Aminen (A) und (B) eingehen, insbesondere Stickstoff und Luft. Wegen der geringen Zahl an Apparaten und des niedrigen Energiebedarfs hat diese Ausführungsform den Vorteil geringer Investitions-und Betriebskosten.

In einer weiteren bevorzugten Ausführungsform des erfindungsgemäßen Verfahrens wird das Absorptionsmedium nach dem in Kontakt bringen mit der Gasmischung auf eine Temperatur erhitzt, bei der eine Phasentrennung in eine wässrige flüssige Phase und eine organische flüssige Phase erfolgt und aus der wässrigen flüssigen Phase durch Reduktion des Drucks und/oder Zufuhr von Wärme saures Gas desorbiert. Die dabei resultierende flüssige Phase wird mit der bei der Phasentrennung erhaltenen organischen flüssigen Phase vereinigt und die vereinigten flüssigen Phasen werden wieder als Absorptionsmedium mit der Gasmischung in Kontakt gebracht. Bei der Abtrennung von CO₂ aus Erdgas oder Synthesegas wird das Erhitzen des beladenen Absorptionsmediums und die Phasentrennung vorzugsweise bei einem Druck durchgeführt, bei dem kein CO₂ desorbiert wird und CO₂ nur aus der bei der Phasentrennung erhaltenen wässrigen Phase desorbiert. Damit lässt sich bei der Abtrennung von CO₂ aus Erdgas der Gehalt an Methan im desorbierten CO₂ gering halten und bei der Abtrennung von CO₂ aus Synthesegas der Gehalt an Wasserstoff und CO im desorbierten CO₂ gering halten.

Das erfindungsgemäße aminhaltige Absorptionsmedium, weist zumindest Wasser als Lösemittel und mindestens ein Amin (A) der Formel (I) 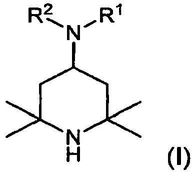 mit R¹ = aliphatischer Rest mit 2 bis 6 Kohlenstoffatomen und mit mindestens einer Aminogruppe,
R² = Wasserstoff, C₁₋₄-Alkylgruppe oder Rest R¹,
auf. Vorzugsweise weist das erfindungsgemäße Absorptionsmedium ein Amin (A) der Formel (I) auf, worin R² Wasserstoff ist.

Bevorzugt weist das erfindungsgemäße Absorptionsmedium ein Amin (A) der Formel (II) 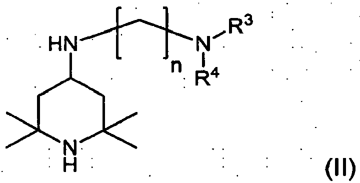 mit R³ = Wasserstoff oder C₁₋₄-Alkylgruppe, bevorzugt Wasserstoff oder Methylgruppe, besonders bevorzugt Methylgruppe,
R⁴ = C₁₋₄-Alkylgruppe, bevorzugt C₁₋₂-Alkylgruppe, besonders bevorzugt Methylgruppe,
n = 2 bis 4, bevorzugt 2 bis 3 und besonders bevorzugt 3,
auf.

Besonders bevorzugt weist das erfindungsgemäße Absorptionsmedium ein Amin (A) der Formel (II) mit R³, R⁴ = Methyl und n = 3 auf.

Die Alkylgruppen können im Rahmen dieser Erfindung substituiert oder unsubstituiert sein, ferner können C₃₋₄-Alkylgruppen linear oder verzweigt sein.

Das erfindungsgemäße Absorptionsmedium weist neben dem Amin (A) ein weiteres Amin (B) der Formel (III) 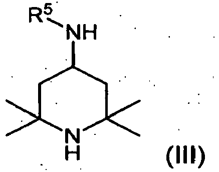 mit R⁵ = C₁₋₆-Alkylgruppe, bevorzugt C₃₋₅-Alkylgruppe, und besonders bevorzugt Butylgruppe,
auf.

Die Alkylgruppen können im Rahmen dieser Erfindung substituiert oder unsubstituiert sein, ferner können C₃₋₆-Alkylgruppen linear oder verzweigt sein. Der Substituent vom Typ R⁵ ist vorzugsweise unsubstituiert, bevorzugt eine unsubstituierte Butylgruppe und besonders bevorzugt eine unsubstituierte n-Butylgruppe.

Das erfindungsgemäße Absorptionsmedium weist zumindest Wasser als Lösemittel auf. Das erfindungsgemäße Absorptionsmedium kann jedoch noch ein weiteres physikalisches Lösemittel (C) enthalten. Dies ist von Vorteil, um die Beladung des Absorptionsmediums mit den sauren Gasen bei hohem Partialdruck des sauren Gases, insbesondere von CO₂, noch weiter zu erhöhen. Auf diese Weise kann der Massenstrom an Absorptionsmedium nochmals reduziert werden kann. Gleichzeitig lässt sich eine energetisch günstigere Regeneration durchführen, indem nicht mehr nur thermisch, sondern alternativ bzw. unterstützend auch durch einen Flash (Druckerniedrigung) regeneriert werden kann.

Die Auswahl der Lösemittels (C) als auch der Gehalt des Lösemittels (C) in dem erfindungsgemäßen Absorptionsmittels richtet sich nach verschiedenen Kriterien, wie beispielsweise Zusammensetzung der zu reinigenden Gasmischung (z.B. Anteile an sauren Komponenten, Anteil an Kohlenwasserstoffen), dem vorliegenden Partialdruck der zu entfernenden sauren Gase, wie beispielsweise CO₂, sowie auch den zu erfüllenden Spezifikationen an das mittels dem erfindungsgemäßen Verfahren gereinigte Gas.

In einer besonderen Ausführungsform des erfindungsgemäßen Absorptionsmittels beträgt der Gehalt an dem Lösemittel (C) von 20 bis 40 Gew.-% bezogen auf das Absorptionsmittels. Diese Ausführungsform eignet sich insbesondere, wenn der Partialdruck des sauren Gases besonders hoch ist, vorzugsweise mindestens 20 bar beträgt, und die Anforderungen an das gereinigte Gas ebenfalls hoch sind, vorzugsweise wenn der Partialdruck des sauren Gases maximal < 10 mbar im gereinigten Gas betragen soll. Auf diese Weise kann die Beladung des Absorptionsmediums mit den sauren Gasen nochmals erhöht werden, dies führt wiederum zu niedrigeren Massenströmen an Absorptionsmedium. Der Anteil an dem Amin (A) beträgt vorzugsweise 10 bis 25 Gew.-% und der Anteil an dem Amin (B) vorzugsweise 5 bis 15 Gew.-% bezogen auf das erfindungsgemäße Absorptionsmedium. Auf diese Weise ist eine Aufreinigung eines Gases möglich mit dem Ziel einen möglichst geringen Partialdruck des sauren Gases zu erzielen.

Als weiteres Lösemittel (C) kann das erfindungsgemäße Absorptionsmedium die aus der Gaswäsche bekannten physikalischen Lösemittel, wie beispielsweise Sulfolan, Propylencarbonat, N-alkylierte Pyrrolidone (z.B. N-Methyl-2-pyrrolidone), und N-alkylierte Piperidone, Dialkylether von Polyethylenglykol und Mischungen daraus, aliphatische Säureamide (z.B. N-Formylmorpholin oder N-Acetylmorpholin). Methylcyanoacetat, aufweisen.

Vorzugsweise weist das erfindungsgemäße Absorptionsmedium
60 bis 80 Gew.-% an Wasser und ggf. Lösemittel (C),
1 bis 40 Gew.-% an dem Amin (A) und
0 bis 39 Gew.-% and dem Amin (B)
auf. Bevorzugt weist das erfindungsgemäße Absorptionsmedium
65 bis 75 Gew.-% an Wasser und ggf. Lösemittel (C),
10 bis 20 Gew.-% an dem Amin (A) und
25 bis 5 Gew.-% and dem Amin (B)
auf.

Besonders bevorzugt weist das erfindungsgemäße Absorptionsmedium

65 bis 75 Gew.-% an Wasser,
10 bis 20 Gew.-% an dem Amin (A) Formel (II) mit R³, R⁴ = Methyl und n = 3 und 25 bis 5 Gew.-% an Amin (B) der Formel (III) mit R⁵ = n-Propyl oder n-Butyl
auf. Mit dieser Zusammensetzung kann für das mit sauren Gasen beladene Absorptionsmittel eine Entmischungstemperatur im Bereich von 85 bis 110 °C eingestellt werden.

Das erfindungsgemäße Absorptionsmedium kann auch Performanceadditive, wie beispielsweise Korrosionsinhibitoren, Aktivatoren, benetzungsfördernde Additive und/oder Entschäumer aufweisen.

Als Korrosionsinhibitoren kann das erfindungsgemäße Absorptionsmedium alle Stoffe aufweisen, die dem Fachmann für Verfahren zur Absorption von CO₂ unter Verwendung von Alkanolamine als geeignete Korrosionsinhibitoren bekannt sind, insbesondere die in US 4,714,597 beschriebenen Korrosionsinhibitoren.

Die Menge an Korrosionsirihibitoren in dem erfindungsgemäßen Absorptionsmedium ist vorzugsweise gegenüber Verfahren gemäß dem Stand der Technik deutlich reduziert, da das erfindungsgemäße Absorptionsmedium gegenüber metallischen Werkstoffen deutlich weniger korrosiv ist als das gemäß dem Stand der Technik häufig verwendete Monoethanolamin.

Als benetzungsförderndes Addditiv weist das erfindungsgemäße Absorptionsmedium vorzugsweise ein oder mehrere Tenside aus der Gruppe der nichtionischen Tenside, zwitterionischen Tenside und kationischen Tenside auf.

Geeignete nichtionische Tenside sind Alkylaminalkoxylate, Amidoamine, Alkanolamide, Alkylphosphinoxide, Alkyl-N glucamide, Alkylglucoside, Gallensäuren, Alkylalkoxylate, Sorbitanester, Sorbitanesterethoxylate, Fettalkohole, Fettsäureethoxylate, Esterethoxylate und Polyethersiloxane.
Geeignete zwitterionischen Tenside sind Betaine, Alkylglycine, Sultaine, Amphopropionate, Amphoacetate, tertiäre Aminoxide und Silicobetaine.

Geeignete kationische Tenside sind quaternäre Ammoniumsalze mit einem oder zwei Substituenten mit 8 bis 20 Kohlenstoffatomen, insbesondere entsprechende Tetraalkylammoniumsalze, Alkylpyridiniumsalze, Esterquats, Diamidoaminquats, Imidazoliniumquats, Alkoxyalkylquats, Benzylquats und Silikonquats.

In einer bevorzugten Ausführungsform des erfindungsgemäßen Absorptionsmediums umfasst das benetzungsfördernde Additiv ein oder mehrere nichtionische Tenside der allgemeinen Formel R(OCH₂CHR')ₘOH mit m von 4 bis 40, worin R ein Alkylrest mit 8 bis 20 Kohlenstoffatomen, ein Alkylarylrest mit 8 bis 20 Kohlenstoffatomen oder ein Polypropylenoxidrest mit 3 bis 40 Propylenoxideinheiten ist und R' Methyl oder vorzugsweise Wasserstoff ist.

In einer weiteren bevorzugten Ausführungsform des erfindungsgemäßen Absorptionsmediums umfasst das benetzungsfördernde Additiv ein Polyether-Polysiloxan-Copolymer, das mehr als 10 Gew.-% [Si(CH₃)₂O]-Einheiten und mehr als 10 Gew.-% [CH₂CHR"-O]-Einheiten enthält, in denen R" Wasserstoff oder Methyl ist. Besonders bevorzugt sind Polyether-Polysiloxan-Copolymere der allgemeinen Formeln (IV) bis (VI):

(CH₃)₃Si-O-[SiR^{a}(CH₃)-O]ₜ-Si(CH₃)₃ (IV)

R^{b}O-Aₚ-[B-A]ₘ-A_{q}-R^{b} (V)

R^{b}O-[A-Z]ₚ-[B-Si(CH₃)₂-Z-O-A-Z]ₘ-B-Si(CH₃)₂[Z-O-A]_{q}O_{1-q}R^{b} (VI)

worin
- A: ein zweiwertiger Rest der Formel -[CH₂CHR^{c}-O]ᵣ- ist,
- B: ein zweiwertiger Rest der Formel -[Si(CH₃)₂-O]ₛ- ist,
- Z: ein zweiwertiger linearer oder verzweigter Alkylenrest mit 2 bis 20 Kohlenstoffatomen und vorzugsweise -(CH₂)₃- ist,
- t: = 1 bis 30 ist,
- m: = 2 bis 100 ist,
- p, q: = 0 oder 1 ist,
- r: = 2 bis 100 ist,
- s: = 2 bis 100, ist,
- R^{a}: von 1 bis 5 der Reste R^{a} Reste der allgemeinen Formel -Z-O-A-R^{b}- sind und die restlichen Reste R^{a} Methyl sind,
- R^{b}: Wasserstoff, ein Alkylrest oder ein aliphatischer oder olefinischer Acylrest mit 1 bis 20 Kohlenstoffatomen ist und
- R^{c}: Wasserstoff oder Methyl ist.

Die benetzungsfördernden Additive sind dem Fachmann bereits aus dem Stand der Technik als Additive für wässrige Lösungen bekannt und können nach aus dem Stand der Technik bekannten Verfahren hergestellt werden.

Das erfindungsgemäße Absorptionsmedium kann sogenannte Aktivatoren aufweisen. Durch den Zusatz von Aktivatoren kann die gewünschte Trennwirkung noch weiter verbessert werden. Als Aktivatoren weist das erfindungsgemäße Absorptionsmedium vorzugsweise primäre oder sekundäre Amine auf, wobei diese keine Struktur gemäß Formel (I) bis (III) aufweisen. Es eignen sich hierfür vorzugsweise Amine, die eine schnelle Kinetik hinsichtlich der Bindung der sauren Gase, insbesondere des CO₂, aufweisen. Bevorzugt weist das erfindungsgemäße Absorptionsmedium Aktivatoren ausgewählt aus Monoethanolamin, Piperazin und 3-(Methylamino)propylamin auf. Das erfindungsgemäße Absorptionsmedium weist vorzugsweise von 0 bis 20 Gew.-% an den Aktivatoren auf.

Eine erfindungsgemäße Vorrichtung zur Abtrennung von sauren Gasen, insbesondere von CO₂, aus einer Gasmischung umfasst eine Absorptionseinheit, eine Desorptionseinheit und ein im Kreislauf geführtes erfindungsgemäßes Absorptionsmedium. Als Absorptionseinheit der erfindungsgemäßen Vorrichtung eignen sich die oben für die Absorption in einem erfindungsgemäßen Verfahren beschriebenen Apparate. Als Desorptionseinheit der erfindungsgemäßen Vorrichtung eignen sich die oben für die Desorption in einem erfindungsgemäßen Verfahren beschriebenen Apparate. Vorzugsweise umfasst die erfindungsgemäße Vorrichtung eine Absorptionseinheit und eine Desorptionseinheit, wie sie dem Fachmann von Vorrichtungen zur Abtrennung von sauren Gasen, insbesondere von CO₂, aus einer Gasmischung unter Verwendung eines Alkanölamins bekannt sind.

Die nachfolgenden Beispiele sollen das erfindungsgemäße Verfahren bzw. das erfindungsgemäße Absorptionsmedium näher erläutern, ohne dass die Erfindung auf diese Ausführungsform beschränkt sein soll.

### Beispiel 1 zur Herstellung von 4-(3-Dimethylamino-propylamino)-2,2,6,6-tetramethylpiperidin (nicht erfindungsgemäß)

1808,9 g (11,65 mol) 2,2,6,6-Tetramethyl-4-piperidinon und 1191,1 g (11,66 mol) N¹,N¹-Dimethyl-1,3-propandiamin werden in einem 4 L Reaktor zusammengegeben und 2 Stunden bei 60 °C gerührt. Anschließend wird das Reaktionswasser im Vakuum abdestilliert. Danach wird die Reaktionslösung in einen Autoklaven überführt und mit 76 g Raney-Nickel versetzt. Der Autoklav wird 3 mal mit je 5 bar Stickstoff gespült. Anschließend wird die Hydrierung durch wiederholtes Aufdrücken von 50 bar Wasserstoff durchgeführt, wobei das Reaktionsgemisch während der gesamten Reaktionszeit kräftig gerührt wird. Anschließend wird das Reaktionsgemisch fraktioniert destilliert. Das Produkt besitzt einen Siedepunkt von 130 °C bei 4,5 mbar. Es konnten 2062 g Produkt mit einer Reinheit von 98,6 % und einer Ausbeute von 72 % der Theorie isoliert werden.

### Beispiele 2 - 12 zur CO₂-Beladung und zum CO₂-Hub

In einer thermostatisierten und mit einer Druckregelung versehenen Apparatur zur Vermessung von Gas-Flüssig-Gleichgewichten wurde ein Absorptionsmedium zusammengesetzt gemäß den Angaben in Tabelle 1 bei konstanter Temperatur vorgelegt und mit gasförmigem Kohlendioxid bei konstantem Druck in Kontakt gebracht, wobei Druck und Temperatur variiert wurden. Jeweils nach Erreichen des Gleichgewichtszustandes wurde der Gehalt an absorbiertem CO₂ in dem beladenen Absorptionsmedium bestimmt und daraus der Beladungsgrad als molares Verhältnis von CO₂ zu Amin im beladenen Absorptionsmedium berechnet. Die untersuchten Temperaturen und Drücke und die dafür bestimmten Beladungsgrade sind in Tabelle 2 zusammengefasst.

**Tabelle 1:**

| **Absorptionsmedium** | **AM 1** | **AM 2** | **AM 3** | **AM 4** | **AM 5** | **AM 6** | **AM 7** |
|---|---|---|---|---|---|---|---|
| | *(in Gew*.*-%)* | | | | | | |
| Wasser | 70 | 50 | 70 | 70 | 70 | 70 | 70 |
| Monoethanolamin | 30 | 0 | 0 | 0 | 0 | 0 | 0 |
| Methyldiethanolamin | 0 | 50 | 0 | 0 | 0 | 0 | 0 |
| n-Butyl-TAD¹ (Amin (B)) | 0 | 0 | 30 | 0 | 15 | 27 | 0 |
| TAT² (Amin (A)) | 0 | 0 | 0 | 30 | 15 | 3 | 0 |
| EAE-TAD³ (Amin (A)) | 0 | 0 | 0 | 0 | 0 | 0 | 30 |
| erfindungsgemäß | nein | nein | nein | ja ⁴ | ja | ja | ja ⁴ |

| | | | | | | | |
|---|---|---|---|---|---|---|---|
| ¹ n-Butyl-TAD: 4-(n-Butylamino)-2,2,6,6-tetramethylpiperidin ² TAT: 4-(3-Dimethylamino-propylamino)-2,2,6,6-tetramethylpiperidin bzw. Triacetontriamin ³ EAE-TAD: 4-(2-Ethylamino-ethylamino)-2,2,6,6-tetramethylpiperidin ⁴ Verfahren erfindungsgemäß, Absorptionsmedium nicht | | | | | | | |

**Tabelle 2:**

| **Beispie l** | **Absorptionsmedium** | **Temperatur** (in °C) | | **Partialdruck CO₂** (in bar) | | **Beladung** (in mol CO₂ / mol Amin) | | **CO₂-Hub** (in mol CO₂ / mol Amin) |
|---|---|---|---|---|---|---|---|---|
| | | **AS⁴** | **DS⁵** | **AS⁴** | **DS⁵** | **AS⁴** | **DS⁵** | |
| **2** | AM 1 | 40 | 120 | 1 | 1 | 0,63 | 0,4 | 0,23 |
| **3** | AM 1 | 40 | 120 | 3 | 1 | 0,7 | 0,4 | 0,3 |
| **4** | AM 2 | 40 | 120 | 1 | 1 | 0,65 | 0,09 | 0,56 |
| **5** | AM 2 | 40 | 120 | 3 | 1 | 0,85 | 0,09 | 0,76 |
| **6** | AM 3 | 40 | 110 | 1 | 1 | 1,2 | 0,3 | 0,8 |
| **7** | AM 3 | 40 | 110 | 3 | 1 | 1,7 | 0,3 | 1,4 |
| **8** | AM 4 | 40 | 110 | 1 | 1 | 1,8 | 0,6 | 1,2 |
| **9** | AM 4 | 40 | 110 | 3 | 1 | 2,2 | 0,6 | 1,6 |
| **10** | AM 5 | 40 | 110 | 1 | 1 | 2,2 | 0,5 | 1,7 |
| **11** | AM 5 | 40 | 110 | 3 | 1 | 2,75 | 0,5 | 2,25 |
| **12** | AM 7 | 40 | 110 | 3 | 1 | 2,2 | 0,65 | 1,55 |

| | | | | | | | | |
|---|---|---|---|---|---|---|---|---|
| ⁴AS: Absorber ⁵DS: Desorber | | | | | | | | |

In der Tabelle 2 sind CO₂-Beiadungen bei unterschiedlichen Temperaturen dargestellt. Die Temperatur von 40 °C entspricht der Beladungstemperatur im Absorber. Dabei können je nach Gaszusammensetzung und Art der Anwendung unterschiedliche Partialdrücke vom CO₂ vorliegen (z.B. p(CO₂) = 1 bar oder p(CO₂) = 3 bar). Die Temperatur von 110 °C bzw. 120 °C entspricht der Desorptionstemperatur, bei der das Lösungsmittel in einem zweiten Apparat (Desorber) wieder regeneriert wird. Üblicherweise wird in einem Druckbereich von 1,5 bis 2,5 bar desorbiert, wobei der CO₂-Partialdruck bei ca. 1 bar liegt. Bei beiden Temperaturen (40 °C und 110 °C bzw. 120 °C) ist das Lösungsmittel unterschiedlich stark mit CO₂ beladen, die Differenz beider Werte entspricht dem CO₂-Hub. Je größer dieser Hub ist, desto geringer ist der Lösungsmittelstrom in der Anlage. Das bedeutet nicht nur geringere Investionskosten, da kleiner Apparate ausreichend sind, sondern hat auch einen großen Einfluss auf die aufzuwendende Desorptionstemperatur im Desorber.

Im Vergleich zu den Verfahren des Stands der Technik (AM 1, AM 2 und AM 3) zeigen die erfindungsgemäßen Verfahren (AM 4, AM 5 und AM 7) deutlich größere CO₂-Hube. Dies kommt einer Ersparnis an Regenerationsenergie gleich, dies wiederum bedeutet eine Minderung der Betriebskosten, beispielsweise hinsichtlich der Dampfmenge für die Regeneration des Lösungsmittels.

Völlig überraschenderweise zeigt das erfindungsgemäße Absörptionsmedium AM 5 - eine Mischung aus n-Butyl-TAD und TAT - nochmals einen deutlich höheren CO₂-Hub als das Absorptionsmedium AM 4, das nur TAT als Amin aufweist. Basierend auf den Ergebnissen bzgl. der Absorptionsmedien AM 3 und AM 4 hätte man für das Beispiel 10 einen CO₂-Hub in dem Wertebereich von 0,8 bis 1,2 erwartet. Dass sich der CO₂-Hub bei dem Beispiele 10 so deutlich verbesserte, war keineswegs vorsehbar und somit völlig überraschend.

Zudem kommt hinzu, dass die ermittelten CO₂-Hube in den Beispielen 8 bis 12 bei einer Regenerationstemperafur von 110°C durchgeführt wurden. Gerade bei den Absorptionsmedien AM 1 und AM 2 sind Regenerationstemperaturen von 120 °C notwendig gewesen. Dies bedeutet, dass sich durch den Einsatz der Absorptionsmedien AM 4, AM 5 und AM 7 die Regenerationsenergie verringert. Daraus ergeben sich für den Betrieb der Anlagen zwei Optionen:
a) Man behält eine Regenerationstemperatur von 120 °C bei und kann so mit einem größeren resultierenden CO₂-Hub auf Grund der geringeren CO₂-Beladung bei 120 °C rechnen. Dies führt zu einer Verringerung der Umlaufrate des Absorptionsmediums. Unter einer Umlaufrate werden im Rahmen dieser Erfindung die Häufigkeit verstanden, die eine definierte Menge an Absorptionsmedium in der erfindungsgemäßen Vorrichtung - einer Absorptions-Desorptionsanlage - im Kreis gefahren werden muss, um eine definierte Menge einer Gasmischung mit einem definierten Gehalt an CO₂ von diesem CO₂ zu befreien.
b) Man wählt eine Regenerationstemperatur von 110°C, dies führt zu Einsparungen an Regenerationsenergie.

### Beispiele 13 - 15 zum Korrosionsverhalten

Die entsprechende elektrochemische Analysemethode (Tafelplotverfahren) wurde gemäß Kladkaew, N. ketal. in Eng. Chem. Res. 2009, 48, 8913-8919 bzw. gemäß ASTM G59-97e1 durchgeführt. Die entsprechenden Ergebnisse sind in der Tabelle 4 zusammengestellt.

**Tabelle 4:**

| **Beispiel** | **Absorptionsmedium begast mit CO₂** | **Korrosionsrate** (in mm/Jahr] |
|---|---|---|
| **13** | AM 1 | 1,991 |
| **14** | AM 3 | 0,223 |
| **15** | AM 5 | 0,18 |

Das erfindungsgemäße Absorptionsmedium AM 5 besitzt im Vergleich zu den Absorptionsmedien gemäß dem Stand der Technik (AM 1 und AM 3) deutlich geringere Korrosionsraten und erhöht damit die Lebensdauer der Anlage bzw. erlaubt den Einsatz günstigerer Werkstoffe, so dass die Investitionskosten verringert werden.

### Beispiele 16 - 22 zum Entmischungsverhalten

In einem druckfesten Glasgefäß wurde ein Absorptionsmedium, zusammengesetzt gemäß den Angaben in Tabelle 5, vorgelegt und bei 20 °C und Atmosphärendruck durch Zugabe von Trockeneis oder durch Durchleiten einer Gasmischung aus 80 Vol% Stickstoff, 6 Vol-% Sauerstoff und 14 Vol-% CO₂ durch das Absorptionsmedium mit CO₂ gesättigt. Das Glasgefäß wurde dann verschtossen und das mit CO₂ beladene Absorptionsmedium wurde in einem Ölbad langsam erhitzt, bis eine Trennung in zwei flüssige Phasen auftrat, die als Trübung der zuvor klaren Mischung erkennbar war. Die so bestimmten Entmischungstemperaturen sind in Tabelle 6 zusammengefasst.

**Tabelle 5:**

| **Absorptionsmedium** | **AM 8** | **AM 9** | **AM 10** | **AM 11** | **AM 12** | **AM 13** | **AM 14** |
|---|---|---|---|---|---|---|---|
| | *(in Gew.-%)* | | | | | | |
| Wasser | 70 | 70 | 70 | 70 | 70 | 65 | 65 |
| n-Butyl-TAD¹ (Amin (B)) | 10 | 0 | 0 | 25 | 15 | 30 | 15 |
| n-Propyl-TAD² (Amin (B)) | 0 | 10 | 0 | 0 | 0 | 0 | 0 |
| Methyl-TAD³ (Amin (B)) | 0 | 0 | 10 | 0 | 0 | 0 | 0 |
| TAT⁴ (Amin (A)) | 20 | 20 | 20 | 5 | 15 | 5 | 20 |
| erfindungsgemäß | ja | ja | ja | ja | ja | ja | ja |

| | | | | | | | |
|---|---|---|---|---|---|---|---|
| ¹ n-Butyl-TAD: 4-(n-Butylamino)-2,2,6,6-tetramethylpiperidin ² n-Propyl-TAD: 4-(n-Propylamino)-2,2,6,6-tetramethylpiperidin ³ Methtyl-TAD: 4-Methylamino-2,2,6,6-tetramethylpiperidin ⁴ TAT: 4-(3-Dimethylamino-propylamino)-2,2,6,6-tetramethylpiperidin bzw. Triacetontriamin | | | | | | | |

**Tabelle 6:**

| **Beispiel** | **Absorptions medium** | **Entmischungstemperatur in °C** | |
|---|---|---|---|
| | | gesättigt bei 0,14 bar CO₂-Partialdruck | gesättigt bei 1 bar CO₂-Partialdruck |
| 16 | AM 8 | n. b. | 107 |
| 17 | AM 9 | n. b. | 115 |
| 18 | AM 10 | n. b. | 119 |
| 19 | AM 11 | 85 | 93 |
| 20 | AM 12 | n. b. | 103 |
| 21 | AM 13 | 95 | 100 |
| 22 | AM 14 | n. b. | 108 |

| | | | |
|---|---|---|---|
| n. b. nicht bestimmt | | | |

Für das Absorptionsmedium AM 4, das nur TAT als Amin enthält, wurde gesättigt bei 1 bar CO₂-Partialdruck auch bei Erhitzen bis 125 °C keine Entmischung beobachtet. Die Beispiele zeigen, dass sich durch die Wahl der Mengenanteile an TAT und n-Alkyl-TAD für das mit sauren Gasen beladene Absorptionsmittel die Entmischungstemperatur einstellen lässt.

## Patentansprüche

1. Verfahren zur Absorption eines sauren Gases aus einer Gasmischung durch in Kontakt bringen der Gasmischung mit einem Absorptionsmedium,
**dadurch gekennzeichnet,**
**dass** ein Absorptionsmedium eingesetzt wird, das zumindest Wasser als Lösemittel und mindestens ein Amin (A) der Formel (I) 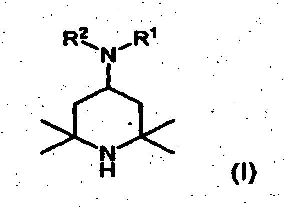 mit R¹ = aliphatischer Rest mit 2 bis 6 Kohlenstoffatomen und mit mindestens einer Aminogruppe,
R² = Wasserstoff, ein C₁₋₄-Alkylgruppe oder ein Rest R¹ aufweist.

2. Verfahren nach Anspruch 1,
**dadurch gekennzeichnet,**
**dass** das Absorptionsmedium mindestens ein Amin (A) der Formel (II) 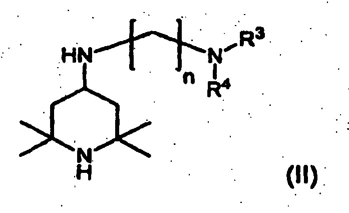 mit R³ = Wasserstoff oder C₁₋₄-Alkylgruppe, bevorzugt Wasserstoff oder Methylgruppe,
R⁴ = C₁₋₄-Alkylgruppe,
n = 2 bis 4,
aufweist.

3. Verfahren nach Anspruch 1 oder 2,
**dadurch gekennzeichnet,**
**dass** das Absorptionsmedium mindestens ein Amin (A) der Formel (II) mit R³, R⁴ = Methyl und n=3 aufweist.

4. Verfahren nach einem der Ansprüche 1 bis 3,
**dadurch gekennzeichnet,**
**dass** das Absorptionsmedium ein weiteres Amin (B) der Formel (III) 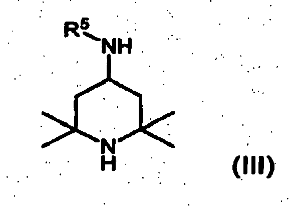 mit R⁵ = C₁-₆-Alkylgruppe, bevorzugt n-Butyl,
aufweist.

5. Verfahren nach einem der Ansprüche 1 bis 4,
**dadurch gekennzeichnet,**
**dass** das eingesetzte Absorptionsmedium noch ein weiteres physikalisches Lösemittel (C) aufweist.

6. Verfahren nach Anspruch 5,
**dadurch gekennzeichnet,**
**dass** das eingesetzte Absorptionsmedium Sulfolan als physikalisches Lösemittel (C) aufweist.

7. Verfahren nach einem der Ansprüche 1 bis 6,
**dadurch gekennzeichnet,**
**dass** das eingesetzte Absorptionsmedium
60 bis 80 Gew.-% an Wasser und ggf. Lösemittel (C),
1 bis 40 Gew.-% an dem Amin (A) und
0 bis 39 Gew.-% and dem Amin (B)
aufweist.

8. Verfahren nach einem der Ansprüche 1 bis 7,
**dadurch gekennzeichnet,**
**dass** das eingesetzte Absorptionsmedium Korrosionsinhibitoren, Aktivatoren, benetzungsfördernde Additive und/oder Entschäumer aufweist.

9. Verfahren nach einem der Ansprüche 1 bis 8,
**dadurch gekennzeichnet,**
**dass** Erdgas oder Synthesegas als Gasmischung eingesetzt wird.

10. Verfahren nach einem der Ansprüche 1 bis 9,
**dadurch gekennzeichnet,**
**dass** der Partialdruck des sauren Gases von 0,1 bar und 20 bar beträgt.

11. Verfahren nach einem der Ansprüche 1 bis 10,
**dadurch gekennzeichnet,**
**dass** die Absorption der sauren Gase bei einer Temperatur von 20 bis 60°C durchgeführt wird.

12. Verfahren nach einem der Ansprüche 1 bis 11,
**dadurch gekennzeichnet,**
**dass** die Desorption der sauren Gase bei einer Temperatur von 60 bis 100°C durchgeführt wird.

13. Verfahren nach einem der Ansprüche 1 bis 12,
**dadurch gekennzeichnet,**
**dass** das saure Gas Kohlendioxid umfasst.

14. Verfahren nach einem der Ansprüche 1 bis 13,
**dadurch gekennzeichnet,**
**dass** das Absorptionsmedium nach dem in Kontakt bringen mit der Gasmischung auf eine Temperatur erhitzt wird, bei der eine Phasentrennung in eine wässrige flüssige Phase und eine organische flüssige Phase erfolgt.

15. Verfahren nach Anspruch 14,
**dadurch gekennzeichnet,**
**dass** aus der wässrigen flüssigen Phase durch Reduktion des Drucks und/oder Zufuhr von Wärme saures Gas desorbiert wird, die dabei resultierende flüssige Phase mit der bei der Phasentrennung erhaltenen organischen flüssigen Phase vereinigt wird und die vereinigten flüssigen Phasen wieder als Absorptionsmedium mit der Gasmischung in Kontakt gebracht wird.

16. Verfahren nach Anspruch 14,
**dadurch gekennzeichnet,**
**dass** nach der Phasentrennung aus der resultierenden zweiphasigen Mischung saures Gas durch Strippen mit einem Gas desorbiert wird.

17. Aminhaltiges Absorptionsmedium,
**dadurch gekennzeichnet,**
**dass** es zumindest Wasser als Lösemittel, mindestens ein Amin (A) der Formel (I) 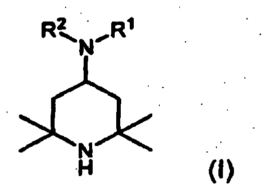 mit R¹ = aliphatischer Rest mit 2 bis 6 Kohlenstoffatomen und mit mindestens einer Aminogruppe,
R² = Wasserstoff, ein C₁₋₄-Alkylgruppe oder ein Rest R¹
und zusätzlich ein weiteres Amin (B) der Formel (III) 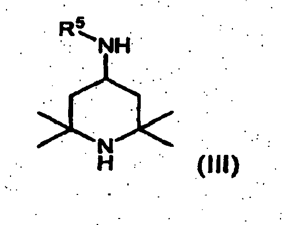 mit R⁵ = C₁₋₆-Alkylgruppe, bevorzugt Butylgruppe,
aufweist.

18. Vorrichtung zur Abtrennung von sauren Gasen aus einer Gasmischung, umfassend eine Absorptionseinheit, eine Desorptionseinheit und ein im Kreislauf geführtes Absorptionsmedium,
**dadurch gekennzeichnet,**
**dass** sie ein Absorptionsmedium nach Anspruch 17 umfasst.

## Claims

1. Process for absorption of an acid gas from a gas mixture by contacting the gas mixture with an absorption medium,
**characterized in that**
an absorption medium is used which comprises at least water as solvent and at least one amine (A) of formula (I) 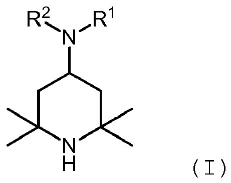 where R¹ = aliphatic radical having 2 to 6 carbon atoms and having at least one amino group,
R² = hydrogen, a C₁₋₄ alkyl group or a radical R¹.

2. Process according to Claim 1,
**characterized in that**
the absorption medium comprises at least one amine (A) of formula (II) 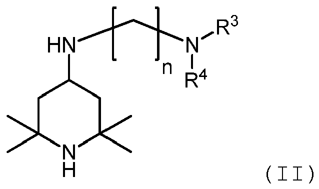 where R³ = hydrogen or C₁₋₄ alkyl group,
preferably hydrogen or methyl group,
R⁴ = C₁₋₄ alkyl group,
n = 2 to 4.

3. Process according to Claim 1 or 2,
**characterized in that**
the absorption medium comprises at least one amine (A) of the formula (II) where R³, R⁴ = methyl and n = 3.

4. Process according to any one of Claims 1 to 3,
**characterized in that**
the absorption medium comprises a further amine (B) of formula (III) 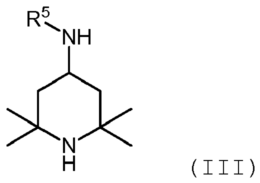 where R⁵ = C₁₋₆ alkyl group, preferably n-butyl.

5. Process according to any one of Claims 1 to 4,
**characterized in that**
the absorption medium used further comprises a further physical solvent (C).

6. Process according to Claim 5,
**characterized in that**
the absorption medium used comprises sulpholane as physical solvent (C).

7. Process according to any one of Claims 1 to 6,
**characterized in that**
the absorption medium used comprises
60 to 80% by weight of water and optionally solvent (C),
1 to 40% by weight of the amine (A) and
0 to 39% by weight of the amine (B).

8. Process according to any one of Claims 1 to 7,
**characterized in that**
the absorption medium used comprises corrosion inhibitors, activators, wetting-promoting additives and/or defoamers.

9. Process according to any one of Claims 1 to 8,
**characterized in that**
natural gas or synthesis gas is used as gas mixture.

10. Process according to any one of Claims 1 to 9,
**characterized in that**
the partial pressure of the acid gas is from 0.1 bar to 20 bar.

11. Process according to any one of Claims 1 to 10,
**characterized in that**
the acid gases are absorbed at a temperature of 20 to 60°C.

12. Process according to any one of Claims 1 to 11,
**characterized in that**
the acid gases are desorbed at a temperature of 60 to 100°C.

13. Process according to any one of Claims 1 to 12,
**characterized in that**,
the acid gas comprises carbon dioxide.

14. Process according to any one of Claims 1 to 13,
**characterized in that**,
the absorption medium, after it has been contacted with the gas mixture, is heated to a temperature at which a phase separation into an aqueous liquid phase and an organic liquid phase occurs.

15. Process according to Claim 14,
**characterized in that**,
acid gas is desorbed from the aqueous liquid phase by reducing the pressure and/or supplying heat, the resulting liquid phase is combined with the organic liquid phase obtained on phase separation and the combined liquid phases are again, as absorption medium, contacted with the gas mixture.

16. Process according to Claim 14,
**characterized in that**,
after the phase separation, acid gas is desorbed from the resulting two-phase mixture by stripping with a gas.

17. Amine-containing absorption medium,
**characterized in that**
it comprises at least water as solvent, at least one amine (A) of formula (I) 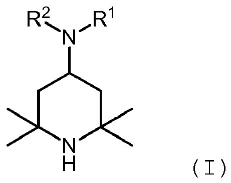 where R¹ = aliphatic radical having 2 to 6 carbon atoms and having at least one amino group,
R² = hydrogen, a C₁₋₄ alkyl group or a radical R¹ and additionally a further amine (B) of formula (III) 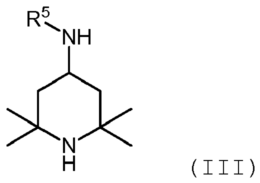 where R⁵ = C₁₋₆ alkyl group, preferably butyl group.

18. Device for separating off acid gases from a gas mixture, said device comprising an absorption unit, a desorption unit and a circulating absorption medium,
**characterized in that**
said device comprises an absorption medium according to Claim 17.

## Revendications

1. Procédé d'absorption d'un gaz acide à partir d'un mélange gazeux par mise en contact du mélange gazeux avec un milieu d'absorption, **caractérisé en ce qu'**on utilise un milieu d'absorption qui présente au moins de l'eau comme solvant et au moins une amine (A) de formule (I) 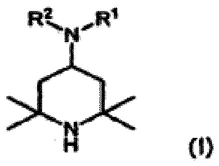 où
R¹ = un radical aliphatique comprenant 2 à 6 atomes de carbone et présentant au moins un groupe amino
R² = hydrogène, un radical C₁₋₄-alkyle ou un radical R¹.

2. Procédé selon la revendication 1, **caractérisé en ce que** le milieu d'absorption présente au moins une amine (A) de formule (II) 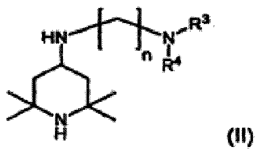 où
R³ = hydrogène ou un groupe C₁₋₄-alkyle, de préférence hydrogène ou un groupe méthyle,
R⁴ = un groupe C₁₋₄-alkyle,
n = 2 à 4.

3. Procédé selon la revendication 1 ou 2, **caractérisé en ce que** le milieu d'absorption présente au moins une amine (A) de formule (II), R³, R⁴ représentant méthyle et n = 3.

4. Procédé selon l'une quelconque des revendications 1 à 3, **caractérisé en ce que** le milieu d'absorption présente une autre amine (B) de formule (III) 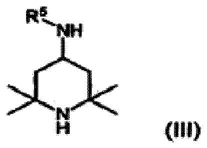 où
R⁵ = un groupe C₁₋₆-alkyle, de préférence n-butyle.

5. Procédé selon l'une quelconque des revendications 1 à 4, **caractérisé en ce que** le milieu d'absorption utilisé présente encore un autre solvant physique (C).

6. Procédé selon la revendication 5, **caractérisé en ce que** le milieu d'absorption utilisé présente du sulfolane comme solvant physique (C).

7. Procédé selon l'une quelconque des revendications 1 à 6,
**caractérisé en ce que** le milieu d'absorption utilisé présente
60 à 80% en poids d'eau et le cas échéant de solvant (C),
1 à 40% en poids de l'amine (A) et
0 à 39% en poids de l'amine (B).

8. Procédé selon l'une quelconque des revendications 1 à 7, **caractérisé en ce que** le milieu d'absorption utilisé présente des inhibiteurs de corrosion, des activateurs, des additifs favorisant le mouillage et/ou des antimousses.

9. Procédé selon l'une quelconque des revendications 1 à 8, **caractérisé en ce qu'**on utilise, comme mélange gazeux, du gaz naturel ou un gaz de synthèse.

10. Procédé selon l'une quelconque des revendications 1 à 9, **caractérisé en ce que** la pression partielle du gaz acide est de 0,1 à 20 bars.

11. Procédé selon l'une quelconque des revendications 1 à 10, **caractérisé en ce que** l'absorption des gaz acides est réalisée à une température de 20 à 60°C.

12. Procédé selon l'une quelconque des revendications 1 à 11, **caractérisé en ce que** la désorption des gaz acides est réalisée à une température de 60 à 100°C.

13. Procédé selon l'une quelconque des revendications 1 à 12, **caractérisé en ce que** le gaz acide contient du dioxyde de carbone.

14. Procédé selon l'une quelconque des revendications 1 à 13, **caractérisé en ce que** le milieu d'absorption est chauffé, après la mise en contact avec le mélange gazeux, à une température à laquelle une séparation de phases en une phase aqueuse liquide et une phase organique liquide se produit.

15. Procédé selon la revendication 14, **caractérisé en ce que** du gaz acide est désorbé de la phase aqueuse liquide par réduction de la pression et/ou introduction de chaleur, la phase liquide ainsi obtenue est réunie avec la phase organique liquide obtenue lors de la séparation de phases et les phases liquides réunies sont à nouveau mises en contact avec le mélange gazeux en tant que milieu d'absorption.

16. Procédé selon la revendication 14, **caractérisé en ce qu'**après la séparation des phases, le gaz acide est désorbé du mélange à deux phases obtenu par rectification à l'aide d'un gaz.

17. Milieu d'absorption contenant une/des amine(s), **caractérisé en ce qu'**il présente au moins de l'eau comme solvant, au moins une amine (A) de formule (I) 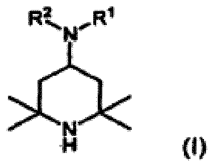 où
R¹ = un radical aliphatique comprenant 2 à 6 atomes de carbone et présentant au moins un groupe amino
R² = hydrogène, un radical C₁₋₄-alkyle ou un radical R¹ et en outre une autre amine (B) de formule (III) 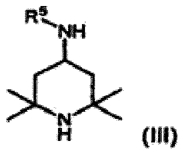 où
R⁵ = un groupe C₁₋₆-alkyle, de préférence n-butyle.

18. Dispositif pour la séparation de gaz acides à partir d'un mélange gazeux, comprenant une unité d'absorption, une unité de désorption et un milieu d'absorption guidé dans un circuit, **caractérisé en ce qu'**il comprend un milieu d'absorption selon la revendication 17.
